# EUROPÄISCHE PATENTSCHRIFT

(11) **EP 1 356 795 B1**
(45) Veröffentlichungstag und Bekanntmachung des Hinweises auf die Patenterteilung: **15.12.2004**
(21) Anmeldenummer: 03005974.5
(22) Anmeldetag: 18.03.2003
(51) Int. Cl.: A61F 2/36

(54) **Set zur Erstellung eines Armierungsimplantates**
Set for constructing a reinforcing implant
Ensemble pour la construction d'un implant de renforcement

(30) Priorität: 23.04.2002 DE 10218801
(43) Veröffentlichungstag der Anmeldung: 29.10.2003
(73) Patentinhaber: ESKA Implants GmbH & Co., 23556 Lübeck (DE)
(72) Erfinder: Grundei, Hans, Dr., 23558 Lübeck (DE); Karpf, P.-M., Prof. Dr., 84028 Landshut (DE)
(74) Vertreter: Fuchs Mehler Weiss & Fritzsche

(56) Entgegenhaltungen:
- EP-A- 0 853 928
- EP-A- 0 878 176
- EP-A- 1 059 070
- DE-A- 3 306 171
- DE-A- 19 629 322
- DE-C- 923 383
- FR-A- 989 341
- FR-E- 59 919
- GB-A- 764 600
- US-A- 2 685 877
- US-A- 5 605 457

## Beschreibung

Die vorliegende Erfindung betrifft ein Set zur Erstellung eines Armierungsimplantates für die Gelenkkugel eines Hüftgelenks.

Dokument DE-C-923 383 stellt den nächsten Stand der Technik dar.

Bei Schädigungen im Hüftgelenksbereich ist man bemüht, so viel natürliches Knochenmaterial zurückzulassen wie möglich. Daher erscheint es bei relativ kleinen Schädigungen, wie etwa einer Hüftgelenkskopfnekrose, als unangemessen, durch Resektionen großer Bereiche einen Ersatz für die dann resezierte Gelenkkugel zu schaffen. Dabei kommen dann üblicherweise sogenannte Langstielendoprothesen zum Einsatz. Bei derartigen Schäden, die den Patienten gleichwohl beeinträchtigen, kommen daher zunehmend sogenannte Schenkelhalsendoprothese zum Einsatz, bei denen der Prothesenstiel lediglich in der Metaphyse und der Diaphyse liegt. Der Einsatz eines derartigen Implantates, wie es beispielsweise bekannt ist aus der EP-A-0 878 176, gestattet, im Falle von Komplikationen nach langer Tragzeit den Rückzug auf die schon genannten und bekannten Langstielendoprothesen. Bei der Implantation der Schenkelhalsendoprothese wird von vornherein eine geringere Resektion des Schenkelhalses vorgenommen als dies bei der Implantation einer Langstielendoprothese der Fall ist. Im Falle des notwendig werdenden Austausches kann nun der Rest des Schenkelhalses reseziert werden in einem Maße wie von vornherein bei Implantationen einer Langstielendoprothese, wodurch die endoprothetische Versorgung der Endoprothesen um einen Zyklus verlängert werden kann.

Schädigungen wie eine Kopfnekrose rechtfertigen selbst eine so vorsichtige Vorgehensweise nicht immer.

Vor diesem Hintergrund ist es die Aufgabe der vorliegenden Erfindung, ein eingangs genanntes Set so auszugestalten, daß die Resektion bei der Implantation auf ein Mindestmaß reduziert werden kann, die Kopfbeschädigungen gleichwohl aber beseitigt werden.

Gelöst wird diese Aufgabe durch das Set mit den Mindestbestandteilen gemäß Anspruch 1. Weitere vorteilhafte Weiterbildungen sind in den Unteransprüchen angegeben.

Als Mindestbestandteile des Sets ist eine der Form der natürlichen Gelenkkugel nachgebildete Kappe vorgesehen, die einen angeformten, von ihrem Polbereich zu ihrer konkaven offenen Seite abragenden Steckkonus aufweist. Darüber hinaus ist eine im Inneren der Kappe umlaufende Fixationshilfe vorgesehen.

Die erfindungsgemäße Kappe wird nach Präparation des natürlichen Gelenkkugelkopfes durch Fräsen auf den verbleibenden Restkopf gesetzt. Hierzu wird nach dem Fräsen des natürlichen Gelenkkopfes in seinem Polbereich eine Bohrung eingebracht, in welche der Steckkonus setzbar ist. Bei einer weiter unten dargestellten Ausführungsform wird in diese Bohrung ein Zapfen gesetzt, welcher mit dem Steckkonus in einem Klemmsitz sitzt.

Die umlaufende Fixationshilfe im Inneren der Kappe sorgt für einen sicheren Sitz der Kappe auf dem Restkopf.

Gemäß einer Ausführungsform ist die Fixationshilfe als umlaufende Nut mit einem dreidimensionalen offenmaschig strukturierten Boden ausgebildet. Die erwähnte Struktur dient dazu, daß Knochentrabekel des umliegenden Knochenmaterials in die Struktur hineinwachsen können, um so für eine stabile Langzeitfixation zu sorgen.

Alternativ hierzu ist eine weitere Ausführungsform, gemäß der die Fixationshilfe ein in eine umlaufende Nut in der Kappe setzbarer Klemmring ist.

Um die Sicherung der Kappe auf dem Restkopf noch zu erhöhen, weist der Klemmring gemäß letzter Ausführungsform gemäß einer vorteilhaften Weiterbildung zumindest teilweise eine dreidimensionale offenmaschige Raumnetzstruktur auf seiner Oberfläche auf. In diese Raumnetzstruktur kann wiederum umgebendes Knochenmaterial herumwachsen und durch diese hindurch, was zu einer hohen Langzeitstabilität führt.

Um diese noch weiter zu fördern, ist gemäß einer weiteren vorteilhaften Ausführungsform vorgesehen, daß der den Boden des Steckkonus umgebende Ringraum mit einer dreidimensionalen offenmaschigen Raumnetzstruktur belegt ist. In diese und durch diese hindurch wachsen Knochentrapekel und sichern den Sitz der Kappe auf dem Restkopf.

Gemäß einer vorteilhaften Weiterbildung umfaßt das Set zusätzlich einen mit dem Steckkonus über eine konische Klemmhülse in einen konischen Klemmsitz bringbaren Zapfen, der schon weiter oben erwähnt worden ist. In diesem Falle wird der Zapfen in die Bohrung im Polbereich der natürlichen Gelenkkugel gesetzt. Vorzugsweise ist die Oberfläche des Zapfens zumindest teilweise mit einer dreidimensionalen offenmaschigen Raumnetzstruktur versehen. Auch in diese und durch diese hindurch wachsen Knochentrabekel, welche für einen sicheren Langzeitsitz des Implantates sorgen.

In einigen Fällen kann es während der Operation zu Komplikationen kommen, welche den Operateur dazu veranlassen, den Rückzug einzuleiten. Wenn die Verbindung zwischen der Kappe und dem Zapfen bereits hergestellt worden ist, ist es in Einzelfällen schwierig, die Kappe wieder vom Zapfen abzuziehen. Eine besondere Ausführungsform sieht daher vor, im Inneren des Steckkonus eine Gewindebohrung vorzusehen, mit der eine Abdrückschraube verschraubbar ist, die durch eine Öffnung im Polbereich der Kappe betätigbar ist. In diesem notwendig werdenden Fall des Abziehens setzt der Operateuer also ein geeignetes Werkzeug durch die Öffnung im Polbereich der Kappe und betätigt die Schraube so lange, bis diese bis zum Ende der Gewindebohrung im Konus gedreht wurde und ein weiteres Verschrauben das Abziehen der Kappe vom Zapfen bewirkt.

Zur besseren Fixation des Zapfens im Restgelenkkopf kann vorgesehen sein, daß die Oberfläche des Zapfens zumindest teilweise mit einer dreidimensionalen offenmaschigen Raumnetzstruktur versehen ist. In diese und durch diese hindurch wachsen Knochentrabekel und sorgen in bewährter Art und Weise für eine Langzeitfixation des Implantates im Knochen.

Besonders vorteilhaft ist das erfindungsgemäße Set, wenn es Kappen mehrerer Größen sowie Zapfen mehrerer Größen umfaßt. Dann kann der Operatuer, nämlich während der Operation die geeigneten Größen auswählen und das Implantat an Ort und Stelle komplettieren.

Die Erfindung wird anhand der Zeichnungsfiguren näher erläutert. Hierbei zeigt:
- Figur 1: eine Schnittansicht durch das Armierungsimplantat, zusammengestellt aus einem ersten Set,
- Figur 2: die Schnittansicht der Kappe gemäß einem anderen Ausführungsbeispiel,
- Figur 3: Schnittansicht durch Zapfen unterschiedlicher Größe,
- Figur 4: die Aufsicht auf einen Klemmring des Sets, und
- Figur 5: die Aufsicht auf die Kappe des Sets.

Nachfolgend bezeichnen gleiche Bezugszeichen gleiche Teile.

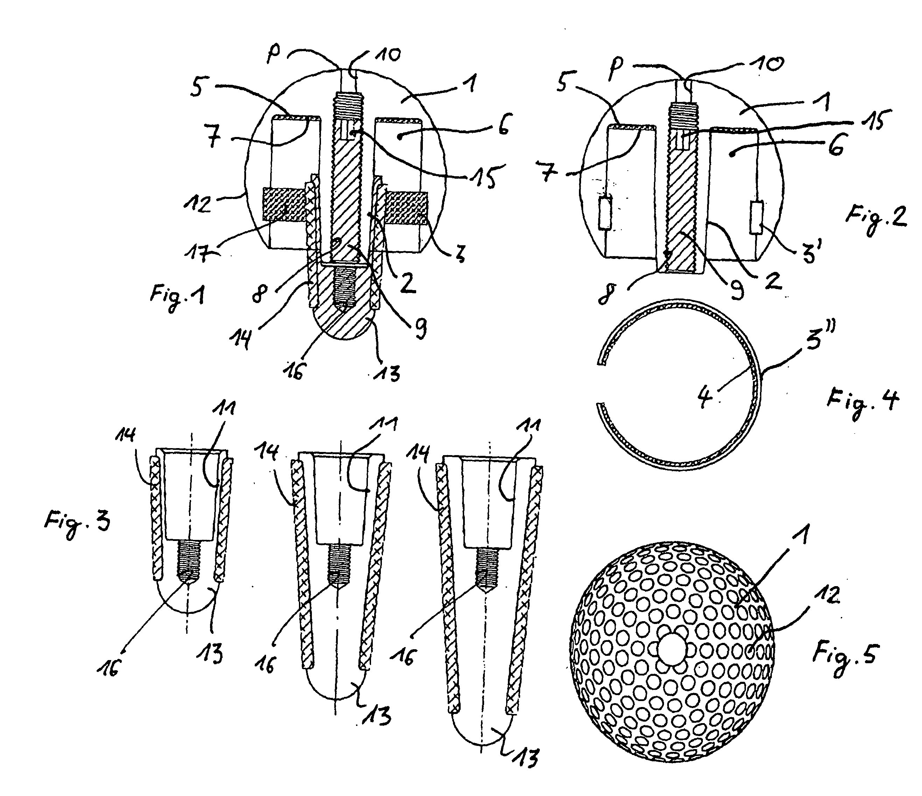

Figur 1 zeigt ein Armierungsimplantat, welches aus einem ersten Set der Erfindung zusammengestellt worden ist. Kernstück des Armierungsimplantates ist die Kappe 1, die aus geeignetem Material wie Metall oder Keramik oder einem Verbundwerkstoff besteht. In ihre Oberfläche sind gleichmäßig verteilt Vertiefungen 12 eingebracht, die untereinander nicht in Verbindung stehen. Die Vertiefungen dienen zur Aufnahme der Gelenkflüssigkeit (Synovia). Die Vertiefungen 12 in Verbindung mit der Synovia führen zu einer Druckverteilung in der Beckenpfanne (nicht dargestellt), die zum Gleiten der Gelenkkugel in der Pfanne führt.

Die Kappe 1 weist in ihrem Inneren einen im Polbereich P angeformten Steckkonus 2 auf. Auf den Steckkonus 2 ist eine konische Klemmhülse 11 gesteckt, die in dem Zapfen 13 eingebracht ist. Die konische Klemmhülse 11 sitzt auf dem Steckkonus 2 in einem konischen Klemmsitz. Der Zapfen 13 ist zumindest bereichsweise mit einer dreidimensionalen offenmaschigen Raumnetzstruktur 14 versehen, in welche und durch welche hindurch Knochentrabekel des umliegenden Knochenmaterials wachsen und zur Erhöhung der sicheren Fixation des Implantates auf dem Gelenkrestkopf sorgt.

Im Inneren der Kappe 1 ist die umlaufende Fixationshilfe 3 angeordnet. Diese besteht in dieser Ausführungsform aus einer umlaufenden Nut, deren Boden mit einer dreidimensionalen offenmaschigen Raumnetzstruktur 17 belegt ist. Auch in diese Struktur wächst zur Fixation des Implantates Knochenmaterial ein.

Es ist ersichtlich, daß der Steckkonus 2 einen Ringraum 6 abgrenzt, welcher den Steckkonus 2 umgibt. Der Boden 5 des Ringraumes 6 ist vorliegend mit einer dreidimensionalen offenmaschigen Raumnetzstruktur 7 belegt, in welche Knochentrabekel einwachsen können und so zu einer stabilen Langzeitfixation beitragen.

Im Inneren des Steckkonus 2 ist eine Gewindebohrung 8 vorgesehen. In dieser Gewindebohrung 8 ist eine Abdrückschraube 9 eingelassen. Durch eine im Polbereich P eingebrachte Öffnung 10 der Kappe kann der Operateur ein geeignetes Werkzeug (nicht dargestellt) in den Werkzeugansatz 15 in der Schraube 9 führen, wobei der Werkzeugansatz 15 vorzugsweise als Innensechskant ausgeführt ist. Erreicht die Abdrückschraube 9 das Ende des Steckkonus 2 und schraubt der Operateur die Schraube 9 weiter, so führt dies zu einem Abheben des Steckkonus 12 aus der konischen Klemmhülse 11 im Zapfen 13.

Figur 2 zeigt ein anderes Ausführungsbeispiel der Kappe 1. Im Unterschied zu der Ausführungsform, wie sie in Figur 1 dargestellt ist, ist die umlaufende Fixationshilfe ausgebildet durch eine eingelassene umlaufende Nut 3'. In diese Nut 3' ist ein Klemmring 3" (Figur 4) einsetzbar. Der Klemmring 3' weist nach innen gewandt eine dreidimensionale offenmaschige Raumnetzstruktur 4 auf, in welche wiederum Knochentrabekel einwachsen können.

Figur 3 zeigt in anschaulicher Weise verschiedene Größen des Zapfens 13. Vorliegend ist am Boden der Steckhülse 11 noch eine Gewindebohrung 16 eingelassen. Die Gewindebohrung 16 dient dazu, nach Abzug der Kappe 1 bei einem notwendig werdenden Entfernen auch des Zapfens 13 aus dem Gelenkrestkopf ein Abzugswerkzeug aufzunehmen, mit welchem dann der Zapfen aus der Ausfräsung im Gelenkrestkopf herausgezogen werden kann.

Figur 5 veranschaulicht nochmals die Verteilung der Vertiefungen 12 auf der Oberfläche der Kappe 1.

## Patentansprüche

1. Set zur Erstellung eines Armierungsimplantates für den Restgelenkkopf eines Hüftgelenks, bestehend mindestens aus einer der Form der Gelenkkopfes nachgebildeten Kappe (1) mit einem angeformten, vom Polbereich (P) zur konkaven offenen Seite abragenden Steckkonus (2) **gekennzeichnet durch** eine im Inneren der Kappe (1) umlaufende Fixationshilfe (3) zur stabilen Langzeitfixation des Implantates auf dem Restgelenkkopf.

2. Set nach Anspruch 1, bei dem die Fixationshilfe (3) eine umlaufende Nut mit einem dreidimensionalen offenmaschigen Boden ist.

3. Set nach Anspruch 1, bei dem die Fixationshilfe (3) ein in eine umlaufende Nut (3') setzbarer Klemmring (3") ist.

4. Set nach Anspruch 3, bei dem der Klemmring (3) zumindest teilweise eine dreidimensionale offenmaschige Raumnetzstruktur auf seiner Oberfläche aufweist.

5. Set nach einem der Ansprüche 1 bis 4, bei dem der den Boden (5) des Steckkonus (2) umgebende Ringraum (6) mit einer dreidimensionalen offenmaschigen Raumnetzstruktur (7) belegt ist.

6. Set nach einem der Ansprüche 1 bis 5, zusätzlich mit einem mit dem Steckkonus (2) über eine konische Klemmhülse (11) in einen konischen Klemmsitz bringbaren Zapfen (13).

7. Set nach einem der Ansprüche 1 bis 6, bei dem im Inneren des Steckkonus (2) eine Gewindebohrung (8) vorgesehen ist, mit der eine Abdrückschraube (9) verschraubt ist, die durch eine Öffnung (10) im Polbereich (P) der Kappe (1) betätigbar ist.

8. Set nach Anspruch 6, bei dem die Oberfläche des Zapfens (13) zumindest teilweise mit einer dreidimensionalen offenmaschigen Raumnetzstruktur (14) versehen ist.

9. Set nach Anspruch 6 oder 8, welches Kappen (1) mehrerer Größen sowie Zapfen (13) mehrerer Größen umfasst.

## Claims

1. Set for constructing a reinforcing implant for the residual condyle of a hip joint, consisting at least of a cap (1) reproducing the shape of the condyle having a moulded-on plug-in cone (2) projecting from the pole region (P) towards the concave open side, **characterised by** an annular fixing aid (3) in the interior of the cap (1) for the stable long-term fixing of the implant on the residual condyle.

2. Set according to claim 1, in which the fixing aid (3) is an annular groove with a three-dimensional open-mesh base.

3. Set according to claim 1, in which the fixing aid (3) is a clamping ring (3") which can be placed in an annular groove (3').

4. Set according to claim 3, in which the clamping ring (3) has at least partly a three-dimensional open-mesh spatial network structure on its surface.

5. Set according to one of claims 1 to 4, in which the annular gap (6) surrounding the base (5) of the plug-in cone (2) is covered by a three-dimensional open-mesh spatial network structure (7).

6. Set according to one of claims 1 to 5, additionally having a pin (13) which can be introduced into a conical press fit via a conical split taper socket (11) by the plug-in cone (2).

7. Set according to one of claims 1 to 6, in which a threaded bore (8), with which a forcing screw (9) is screwed, which can be actuated through an opening (10) in the pole region (P) of the cap (1), is provided in the interior of the plug-in cone (2).

8. Set according to claim 6, in which the surface of the pin (13) is provided at least partly with a three-dimensional open-mesh spatial network structure (14).

9. Set according to claim 6 or 8, which comprises caps (1) of several sizes and pins (13) of several sizes.

## Revendications

1. Ensemble de réalisation d'un implant d'armature pour le reste de condyle d'une articulation de la hanche, consistant au moins en un chapeau (1) de forme selon le condyle, avec un cône à enficher (2) venu de moulage, dépassant de la zone du pôle (P) vers le côté ouvert concave, **caractérisé par** un auxiliaire de fixation (3) périphérique à l'intérieur du chapeau (1) pour une fixation stable de longue durée de l'implant sur le reste de condyle.

2. Ensemble selon la revendication 1, dans lequel l'auxiliaire de fixation (3) est une rainure circulaire avec un fond tridimensionnel à mailles ouvertes.

3. Ensemble selon la revendication 1, dans lequel l'auxiliaire de fixation (3) est un anneau de blocage (3") pouvant être introduit dans une rainure circulaire (3').

4. Ensemble selon la revendication 3, dans lequel l'anneau de blocage (3") présente, au moins en partie, à sa surface, une structure tridimensionnelle en réseau spatial à mailles ouvertes.

5. Ensemble selon l'une quelconque des revendications 1 à 4, dans lequel le fond (5) de l'espace annulaire (6) entourant le fond (5) du cône à enficher (2) est garni d'une structure tridimensionnelle en réseau spatial à mailles ouvertes (7).

6. Ensemble selon l'une quelconque des revendications 1 à 5, avec en plus une cheville (13) pouvant être amenée dans un ajustement conique serré avec le cône à enficher (2) par l'intermédiaire d'une douille de serrage conique (11).

7. Ensemble selon l'une quelconque des revendications 1 à 6, dans lequel un trou taraudé (8) est prévu à l'intérieur du cône à enficher (2), avec lequel est vissée une vis à chasser (9), qui peut être actionnée par une ouverture (10) dans la zone du pôle (P) du chapeau (1).

8. Ensemble selon la revendication 6, dans lequel la surface de la cheville (13) est dotée, au moins en partie, d'une structure tridimensionnelle en réseau spatial à mailles ouvertes (14).

9. Ensemble selon la revendication 6 ou 8, comprenant des chapeaux (1) de plusieurs tailles, ainsi que des chevilles (13) de plusieurs tailles.
